# EUROPEAN PATENT APPLICATION

(11) **EP 1 854 409 A2**
(43) Date of publication of application: **14.11.2007**
(21) Application number: 07016302.7
(22) Date of filing: 12.11.2004
(51) Int. Cl.: A61B 5/155, A61B 5/15, A61B 10/00

(54) **Lancing device with depth-control mechanism**

(30) Priority: 12.11.2003 US 519232 P
(62) Divisional of application: 04818705.8
(71) Applicant: Facet Technologies, LLC, Marietta, GA 30067 (US)
(72) Inventor: The designation of the inventor has not yet been filed
(74) Representative: Harland, Linda Jane

(57) **Abstract**

A lancing device comprises a multi-lancet cartridge having a plurality of lancets, a housing for receiving the cartridge having an exterior sidewall defining a lancing opening and a drive mechanism operable to propel an active one of the lancets through a lancing stroke to penetrate the skin. An advancing mechanism is operable to sequentially advance the lancets of the multi-lancet cartridge sequentially into engagement with the drive mechanism. A depth control mechanism provides adjustment of the depth of penetration into the skin of the active lancet. The depth control mechanism may include a wall rotationally mounted in the housing and interposed between the cartridge and the housing sidewall and defining a plurality of depth-control openings. The wall is rotatable relative to housing and the cartridge to a plurality of positions in each of which a different one of the depth-control openings is aligned with the active lancet. The selected depth-control opening adjusts the depth of penetration of the active lancet on its lancing stroke.

## Description

### Technical Field

The present invention relates generally to medical devices and procedures, and to related methods of manufacture; and more particularly to lancing devices for the collection and/or analysis of samples of blood or other bodily fluids, and to replaceable multi-lancet cartridge assemblies for use in connection with such lancing devices.

### Background of the Invention

Many medical procedures require puncturing of the skin, and sometimes underlying tissues, of an animal or human subject. For example, a sharp lancet tip is commonly used to puncture the subject's skin at a lancing site to obtain a sample of blood, interstitial fluid or other body fluid, as for example in blood glucose monitoring by diabetics and in blood typing and screening applications.

In some instances, a person must periodically sample their blood for multiple testing throughout the day or week. Because re-use of a lancet can result in infection or spread of blood borne contaminants, persons requiring repeated testing often must carry multiple lancets with them, which are separately loaded into a lancing device for each sampling. This can be inconvenient and may lead to reduced compliance with a prescribed test regimen. Additionally, the need for repeated loading and unloading of lancets into a lancing-device can be quite conspicuous and distracting to others when done in public, resulting in reduced compliance by some users.

Accordingly, improved lancing devices have been developed, which are capable of carrying out multiple sampling procedures without the need for separately loading individual lancets. For example, PCT International Publication No. WO 03/071940 A1 (International Application No. PCT/US03/05159, filed 20 February 2003), which is incorporated herein by reference, discloses a lancing device including a replaceable multi-lancet cartridge. Such lancing devices provide considerable advantage over single-lancet devices, warranting continued development of related technologies capable of providing further improved convenience and discretion in use.

It is to the provision of an improved sampling device and cartridge meeting these and other needs that the present invention is primarily directed.

### Summary of the Invention

In accordance with the invention there is provided a lancing device for use in combination with a multi-lancet cartridge comprising a plurality of lancets for lancing skin, the lancing device comprising a housing that receives therein the multi-lancet cartridge, the housing being disc-shaped and having a curved exterior sidewall defining a lancing opening, a drive mechanism operable to propel an active one of the lancets through a lancing stroke to penetrate the skin, an advancing mechanism operable to sequentially advance the lancets of the multi-lancet cartridge sequentially into engagement with the drive mechanism, and a depth-control mechanism providing adjustment of a depth of the penetration into the skin of the active lancet during its lancing stroke, the depth-control mechanism including a wall that is curved, rotationally mounted to the housing, interposed between the cartridge the housing sidewall, and defines a plurality of depth-control openings, wherein the wall is rotatable relative to the housing and cartridge to a plurality of positions, wherein in each of the positions a different one of the depth-control openings is aligned with the active lancet and the lancing opening of the housing for receiving the active lancet therethrough during its lancing stroke.

### Brief Description of the Drawing Figures

**FIGURE 1** is a perspective schematic view of a lancing device and a cartridge according to an example embodiment of the invention

**FIGURES 2a** and **2b** are a perspective and an assembly view of a multi-lancet cartridge according to an example embodiment of the invention.

**FIGURE 3** is a perspective view of a lancing device according to another example embodiment of the invention, having a cartridge loaded therein, and with an upper portion of the housing removed for clarity.

**FIGURE 4** shows the drive mechanism portion of a lancing device according to another example embodiment of the invention, including a ratchet mechanism for preventing partial cocking.

**FIGURES 5a - 5c** show a torsion spring mechanism for advancing through sequential lancets of a lancet cartridge, and to prevent reverse movement and/or partial advancement between lancets, in a lancing device according to another example embodiment of the invention.

**FIGURE 6** shows a rotating advancer mechanism of a lancing device for advancing through sequential lancets of a lancet cartridge, according to an example embodiment of the invention.

**FIGURES 7a** and **7b** show a leaf spring mechanism for indexing and advancement through sequential lancets of a lancet cartridge, and to prevent reverse movement of the cartridge, according to another example embodiment of the invention.

**FIGURES 8a** and **8b** show an indexer arm mechanism for engagement with cooperating features of a lancet cartridge to index and advance through sequential lancets of a lancet cartridge, and to prevent reverse movement of the cartridge, according to another example embodiment of the invention.

**FIGURE 9** shows an embodiment of a lancet cartridge having a lancet-retaining ring for preventing lancets from moving radially until advanced into a firing position.

**FIGURES 10a** and **10b** show an embodiment of a lancet cartridge having a plurality of molded cantilevers in the cartridge top cover for lancet retention.

**FIGURES 11a** and **11b** show a cartridge embodiment having a flat stamped spring ring with a plurality of individual spring loops for biasing removed lancet caps out of the lancet's travel path.

**FIGURE 12** shows a cartridge embodiment having transversely-biased spring members for biasing removed lancet caps out of the lancet's travel path.

**FIGURES 13a-13c** show different embodiments of spring clip members for retraction of the protective endcap of each individual lancet.

**FIGURE 14** shows a cartridge embodiment having lancet hold-down snaps for retaining individual lancets.

**FIGURE 15** shows an anti-rotation interlock mechanism for locking the cartridge when the drive mechanism is cocked and allowing advancement of the cartridge after firing, according to an example embodiment of the invention.

**FIGURES 16a** and **16b** show an anti-rotation interlock mechanism according to another embodiment of the invention.

**FIGURE 17** shows another embodiment of a lancing device according to the present invention, having a spring-biased plunger for indexing and advancement through sequential lancets of a lancet cartridge, and to prevent reverse movement of the cartridge.

**FIGURE 18** shows another embodiment of a lancing device, including a cantilevered spring arm for preventing double-cocking of the lancing device.

**FIGURE 19** shows a lancet drive mechanism of a lancing device according to an example embodiment of the invention, having an in-line configuration of the drive and return springs.

**FIGURE 20** shows a lancet drive mechanism of a lancing device according to another embodiment of the invention, having the drive and return springs in a laterally-offset configuration.

**FIGURE 21** shows a trigger mechanism for a lancing device according to an example embodiment of the invention, having a transversely-sliding cage for engaging and releasing the drive piston.

**FIGURES 22 - 26** show trigger mechanisms according to alternate embodiments of the invention, having shutter mechanisms for engaging and releasing the drive piston.

**FIGURES 27a** and **27b** show another embodiment of a trigger mechanism for a lancing device, having a flexing trigger button arm with a shutter aperture at its free end.

**FIGURE 28** shows another embodiment of a trigger mechanism for a lancing device, having a flexing trigger button element integrally molded with the.drive piston.

**FIGURES 29a** and **29b** show another embodiment of a trigger mechanism for a lancing device, having a hinged trigger button.

**FIGURE 30** shows a rotational depth-control mechanism for a lancing device, according to an example embodiment of the invention.

**FIGURE 31** shows a pivotal depth-control member for a lancing device.

**FIGURE 32** shows a translational depth-control mechanism for a lancing device.

**FIGURE 33** shows a screw-driven translational depth-control member for a lancing device.

**FIGURES 34-36** show several alternate embodiments of a rotating disk depth-control member for a lancing device.

**FIGURE 37** shows a cam slot driven translational depth-control mechanism for a lancing device.

**FIGURE 38** shows a screw slot driven translational depth-control mechanism for a lancing device.

**FIGURE 39** shows a geared translational depth-control, mechanism for a lancing device.

**FIGURE 40** shows a multi-shutter depth-control mechanism for a lancing device.

**FIGURE 41** shows alternate forms of direct and indirect actuation of depth-control mechanisms for a lancing device.

**FIGURES 42** and **43** show a alternate forms of pivotal depth-control mechanisms for a lancing device.

**FIGURE 44** shows an extensible iris depth-control mechanism for a lancing device.

**FIGURE 45** shows a pivoting panel depth-control mechanism for a lancing device.

**FIGURE 46** shows a slotted depth-control mechanism for a lancing device.

**FIGURE 47** shows a flexible strip depth-control mechanism for a lancing device.

**FIGURE 48** shows a gear-driven translational depth-control mechanism fora lancing device.

**FIGURE 49** shows a removable member depth-control mechanism for a lancing device.

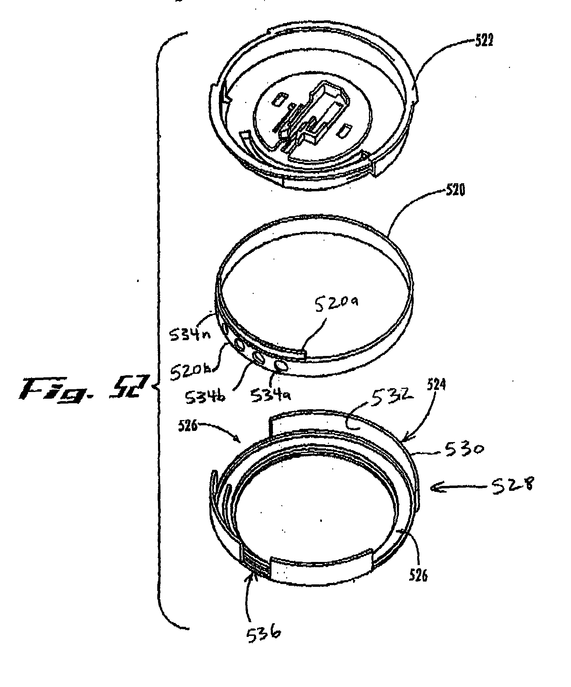

**FIGURES 50-53** show alternate embodiments of rotational member depth-control mechanisms for a lancing device.

### Detailed Description of Example Embodiments

The present Invention may be understood more readily by reference to the following detailed description of the invention taken In connection with the accompanying drawing figures, which form a part of this disclosure. It is to be understood that this invention is not limited to the specific devices, methods, conditions or parameters described and/or shown herein, and that the terminology used herein is for the purpose of describing particular embodiments by way of example only and is not intended to be limiting of the claimed invention. Also, as used in the specification including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment.

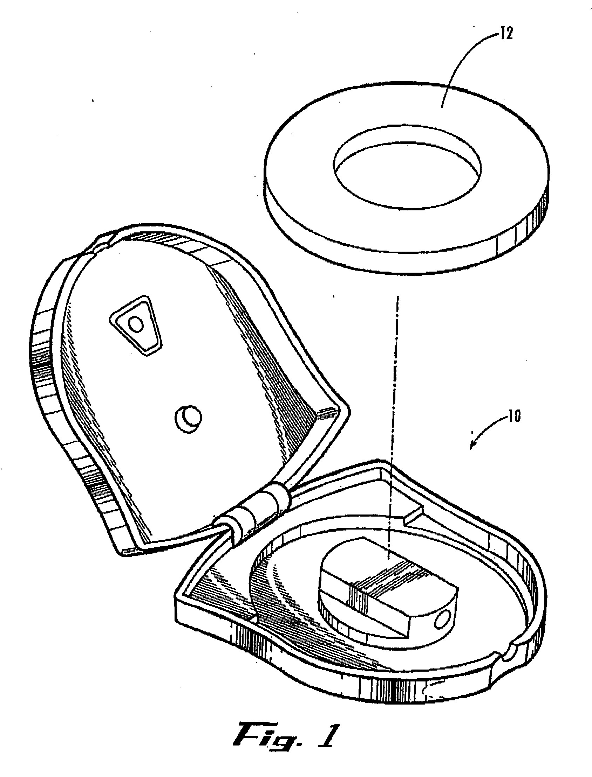

In its various embodiments, the present invention provides an improved lancing device **10**, preferably for use in combination with a replaceable multi-lancet cartridge **12**, as shown schematically for example in **FIGURE 1**. While the improvements of the present invention are adaptable for application in connection with various forms of multi-lancet lancing devices, PCT International Publication No. WO 03/071940 A1, incorporated herein by reference, shows an example form of a multi-lancet lancing device to which the improvements of the present invention are of potential application. It will be recognized that the improvements disclosed herein are of individual advantage, or can be used in combination with one another. In general, the lancing device **10** of the present invention comprises a housing defining a chamber for receiving the cartridge; a drive mechanism for propelling an active lancet of the cartridge through a lancing stroke, from a retracted position within the cartridge to an advanced position wherein a sharp tip of the active lancet projects through a lancet opening in the housing to pierce the subject's skin at an intended lancing site; a charging mechanism for energizing the drive mechanism; and an advancing mechanism for sequentially advancing lancets of the cartridge into and through the active position. Various of these mechanisms can be combined; for example, a single mechanism optionally serves to energize the drive mechanism and simultaneously or sequentially advance the cartridge.

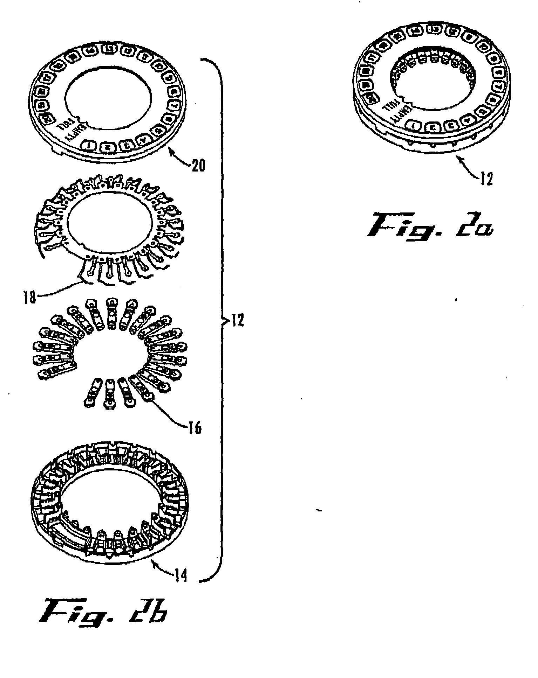

**FIGURE 2** shows a replaceable multi-lancet cartridge **12** according to an example embodiment of the present invention. The cartridge **12** preferably comprises a lancet carrier or base **14,** preferably defining a plurality of lancet guide tracks through which individual lancets traverse upon actuation, defining their respective lancing strokes. The cartridge **12** preferably comprises a plurality of lancets **16**, each slideable within a corresponding guide track. Each lancet **16** preferably comprises a lancet body having a sharp lancet tip projecting therefrom, and a removable protective endcap covering the sharp lancet tip. One or more biasing members **18** are preferably provided, for moving protective endcaps removed from the lancets out of the path of travel of each active lancet as it traverses its lancing stroke. A cover **20** preferably overlies the lancets and couples with the base **14** to provide an enclosure. The cover optionally comprises numerical or other indicia, observable from the exterior of the lancing device when in use, showing which lancet is in use, and/or how many lancets remain for use, and/or that the device is full (unused cartridge) and/or empty (fully used cartridge).

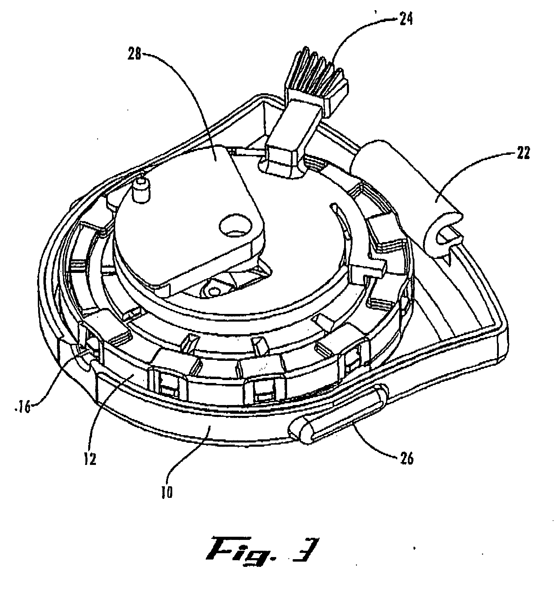

**FIGURE 3** shows a lancing device **10,** with its upper housing half-shell removed for clarity, having a multi-lancet cartridge **12** installed therein. The upper housing half-shell would be pivotally connected to the lower housing half-shell by a hinge coupling **22**, as partially depicted. An arm **24** is pivotally operable to advance the cartridge to bring sequential lancets **16** of the cartridge into the active position, and to energize the drive mechanism and de-cap the active lancet. An activation button or trigger **26** releases the drive mechanism to propel the active lancet through its lancing stroke. A depth-control mechanism **28** is provided, to allow the user to selectively adjust the penetration depth of the lancet.

### Advancing and Indexing

Example embodiments of the lancing device of the present invention preferably include one or more mechanisms for advancing through sequential lancets of a muiti-lancet cartridge or lancet array, for indexing the advancement to prevent partial advancement of the cartridge to a position between lancets wherein no lancet is in the active position, to prevent double-cocking the device and thereby advancing a lancet into and through the active position without using that lancet, and/or to prevent reverse operation and potential re-use of a lancet.

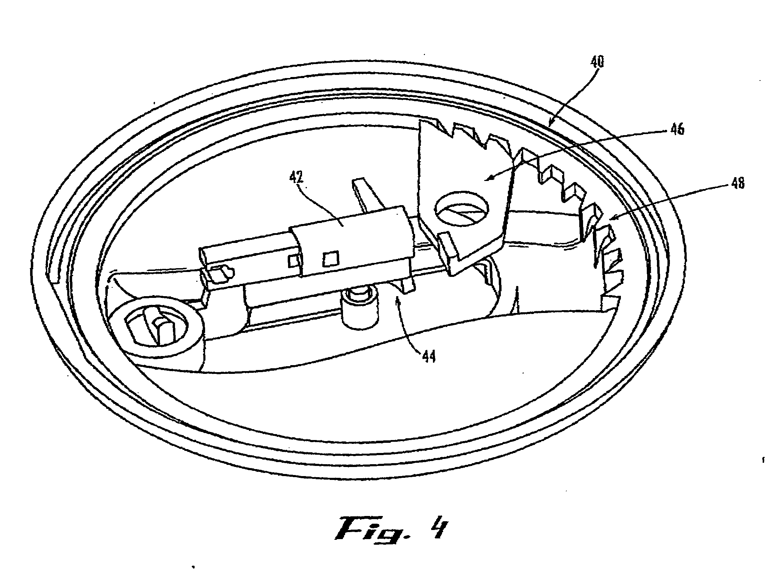

For example, **FIGURE 4** shows an anti-reverse mechanism for a multi-lancet lancing device according to an example embodiment of the present invention. A ratchet and pawl mechanism **40** allows the device to be advanced through sequential lancets of a lancet array in or on a lancet cartridge, and prevents reverse movement and/or partial advancement between lancets. As the drive piston **42** is energized, a contact surface **44** preferably engages the pawl **46** at the limit of its travel, releasing the ratchet **48** to allow the advancing and/or arming mechanism to return to its normal or default position. In this manner, the advancing mechanism operates in a back-and-forth manner, wherein the user actuates the mechanism in a first or forward direction in engagement with the cartridge to advance the cartridge to the next lancet, and in a second or reverse direction out of engagement with the cartridge to return the mechanism to its original postion. In alternate embodiments, the advancing mechanism is actuated continuously in one direction (i.e., clockwise or counter-clockwise) through all the lancets of the cartridge, without the need for any return stroke.

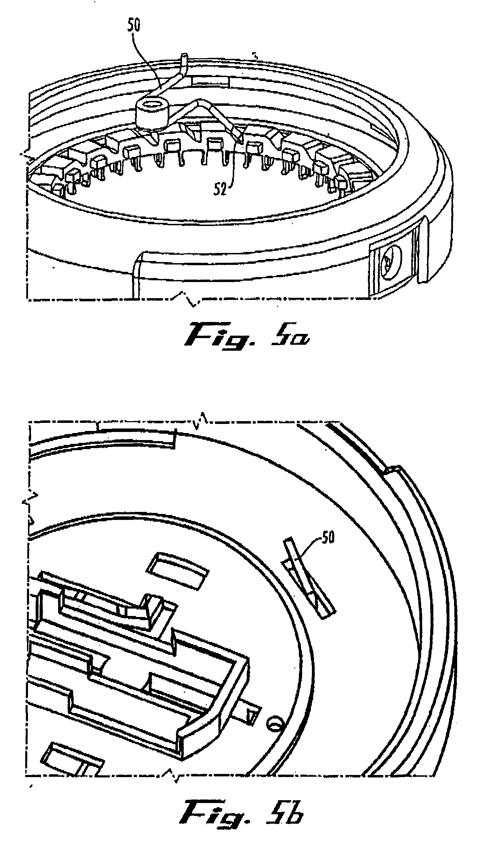

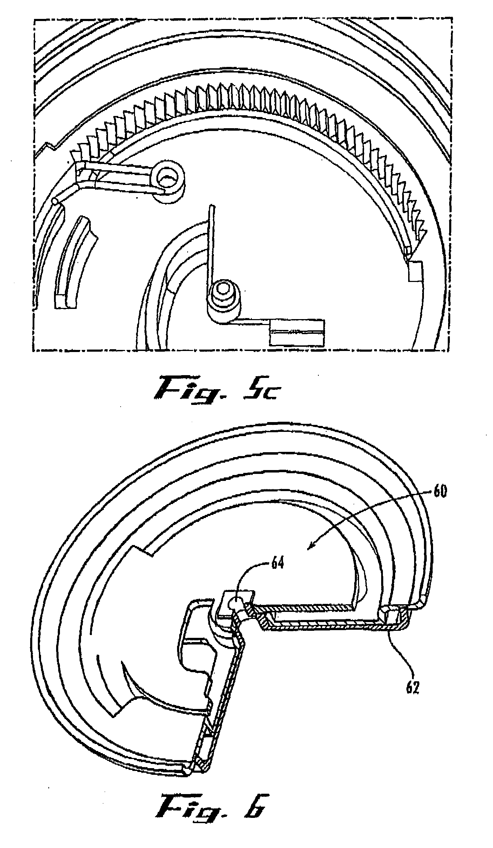

**FIGURES 5a - 5c** show another embodiment of an anti-reverse mechanism, including a torsion spring **50** for engagement with cooperating detents **52** in the cartridge, forming a ratchet mechanism to allow the cartridge to advance through sequential lancets, and to prevent reverse movement of the cartridge, The mechanism preferably also provides indexing of the advancement through sequential lancets, to prevent partial advancement between lancets. In alternate embodiments, a frictional clutch mechanism is provided to prevent reverse movement of the cartridge.

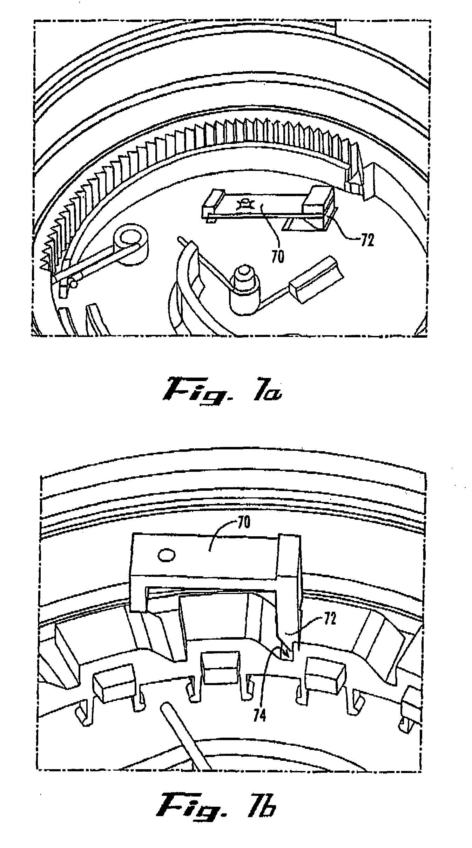

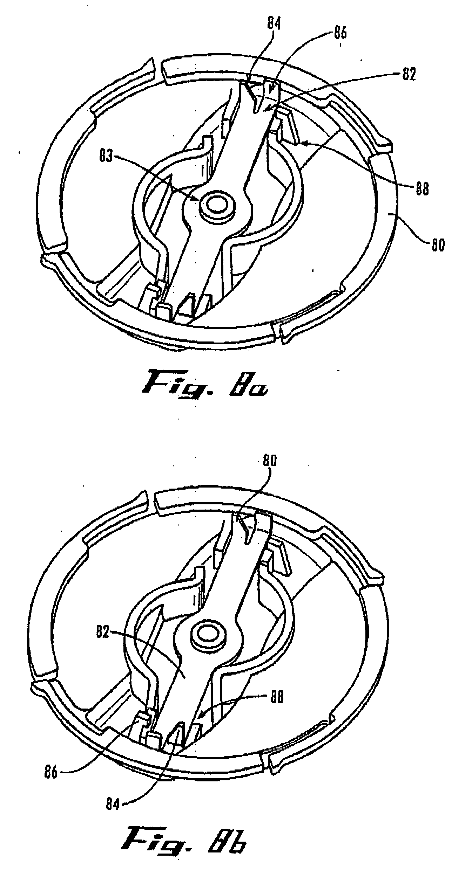

**FIGURE 6** shows a rotational advancing mechanism **60** for a multi-lancet lancing device according to an example embodiment of the invention. The advancing mechanism includes an external rotating advancer knob **62**, coupled to an internal advancer **64** that engages the Cartridge to advance through sequential lancets when the user rotationally actuates the knob.

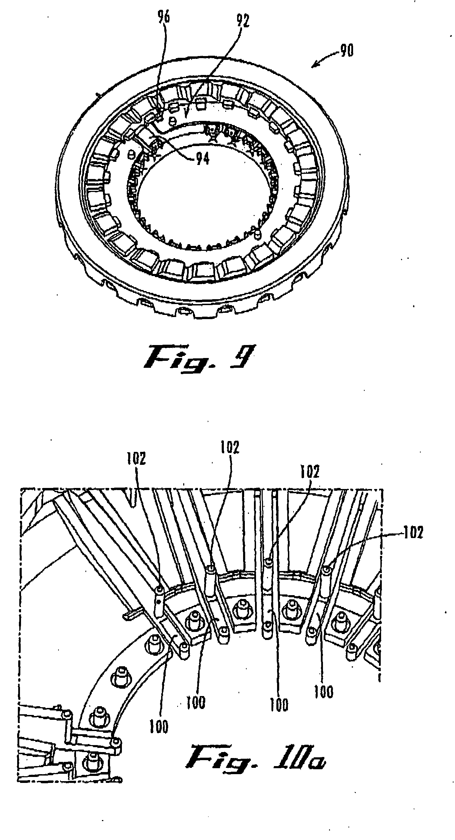

**FIGURES 7a** and **7b** show an anti-reverse mechanism for a multi-lancet lancing device according to an example embodiment of the invention, including a leaf spring **70** having a tooth or finger **72** for engagement with cooperating detents **74** in the lancet cartridge for indexing and advancement through sequential lancets of the cartridge, and to prevent reverse movement of the cartridge.

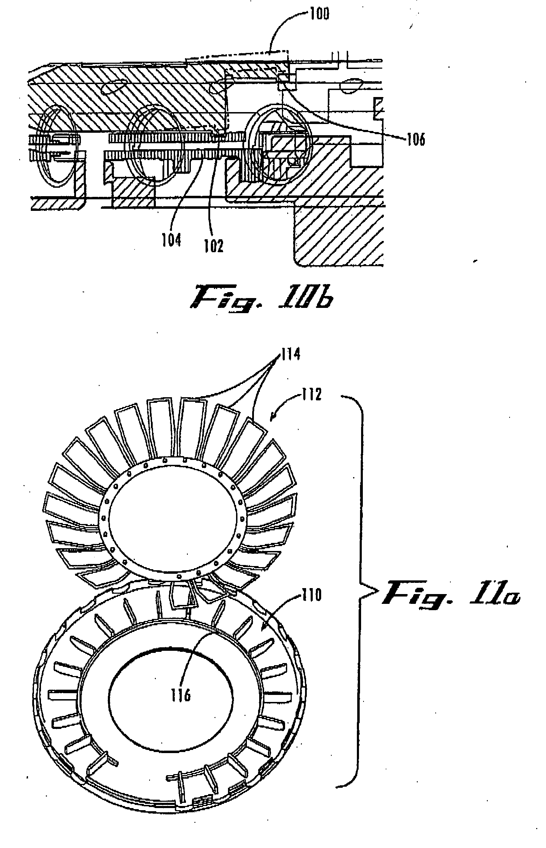

**FIGURES 8a** and **8b** show an advancing knob **80** having an indexer arm **82** mounted, as by heat staking or adhesive, to a central hub **83** of the knob. The arm **82** is preferably formed as a double-ended flexible metal member. Each end of the arm preferably includes a first angled finger **84** for engagement with cooperating features of the lancet cartridge to index and advance the cartridge through sequential lancets, and a second angled finger **86** for tracking a cam surface within the lancing device housing to flex the indexer arm **82** into and out of engagement with the cartridge at the appropriate location of the actuation sequence. This mechanism allows the advancing knob **80** to be rotationally actuated in a single direction, 180° per index step, without the need for a return stroke. Symmetrical guidance and advancing features **88** are preferably arranged adjacent the angled fingers at either end.

### Lancet and Endcap Retention

Example embodiments of the lancing device of the present invention preferably also comprise features for retaining lancets in position in or on a multi-lancet cartridge until advanced into engagement with the drive mechanism at the active lancet position. In this manner, inadvertent discharge of lancets from a cartridge, jamming, and/or noise due to rattling of loose lancets in a cartridge is prevented. The lancing device preferably also includes features for displacing protective endcaps that are removed from the active lancet out of the lancet's path of travel along the lancing stroke, and retaining the removed endcaps.

For example, **FIGURE 9** shows a multi-lancet cartridge **90** according to an example embodiment of the invention having a lancet-retaining ring **92** for preventing lancets from moving radially until advanced into a firing or "active" position beneath a split or open segment of the ring **92**. A retaining pin or projection **94** can be provided on each lancet for engagement with a cooperating track, channel or edge of the ring. The ring **92** optionally includes a limit member **96** to prevent further advancement of the lancet cartridge after it has been advanced through all of the lancets, to prevent re-use of lancets.

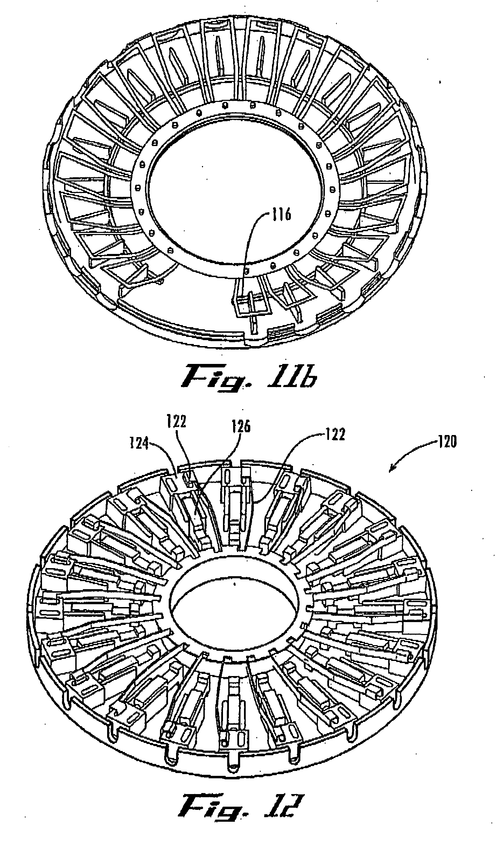

**FIGURES 10a** and **10b** show an embodiment of the invention having a plurality of molded cantilevers **100** formed in a top cover portion of the cartridge for retaining lancets in position in the cartridge until the lancet is advanced into the active position. A lancet retaining pin **102** on each cantilever engages a respective lancet **104** to secure it in position, until the lancet is advanced into the active position, wherein a lifter **106** engages the cantilever **100** to flex it out of engagement with the active lancet **104,** releasing the lancet for firing.

**FIGURES 11a** and **11b** show a cartridge cover **110** having a fiat stamped spring ring **112** with a plurality of individual spring loops **114** mounted thereon. The spring ring **112** is preferably attached to the cover **110** by press-fitting holes of the spring ring onto Tinnerman-style pins, and a raised ring or flange **116** projecting from the cover causes each of spring loops **114** to be outwardly deflected during assembly to preload the loops to bias the endcaps out of the plane of the lancet array upon removal from the lancet at the active position. The provision of a flat spring ring mounted in this manner has been found to provide easier and less expensive manufacture and handling during assembly than forming a pre-bent spring member. For further advantage during assembly, a plurality of such spring rings may be provided in a connected strip, or in a stacked array, for automated dispensing during production.

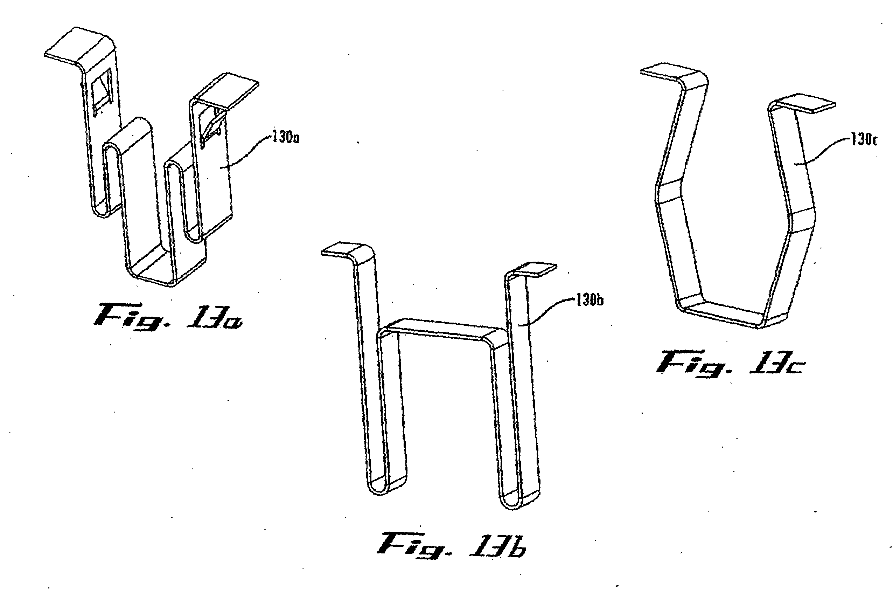

**FIGURE 12** shows a cartridge **120** having a transversely-biased spring member **122** engaging the protective cap **124** of each lancet **126**, for lateral, in-plane removal of the lancet cap. The spring member **122** is pre-loaded upon assembly, so that upon removal of the cap **124** from a lancet **126**, the spring member pulls the cap to the side, out of the active lancet's path of travel. This configuration allows removed endcaps to be stored in the same plane as the lancet array, yet still out of the active lancet's path of travel, thereby reducing the necessary cartridge thickness as compared to cartridges that store the removed endcaps in a well or recess beneath or above the plane of the lancets.

**FIGURES 13a-13c** show alternate embodiments of individual spring clips **130,** for use in a multi-lancet cartridge providing a separate spring clip member for engagement and retraction of the protective endcap of each individual lancet. In further alternate embodiments, two or more (for example, 2, 4, 5 or 10) connected spring clips are formed as a spring segment or strip, and multiple segments are installed with a spring clip engaging each lancet endcap.

**FIGURE 14** shows a portion of a cartridge according to another embodiment of the invention, having resilient lancet hold-down snaps **140** for holding the lancets in place during assembly and use. The lancet is able to slide freely between the hold-down snaps, but the snaps prevent the lancets from being displaced from the cartridge. The snaps optionally also serve as directional guides for the lancet's travel during firing.

### Cartridge Alignment

Example embodiments of the lancing device of the present invention preferably also include anti-rotation interlock features for fixing the cartridge in position when the drive mechanism of the device is charged (i.e., in its cocked configuration). In this manner, proper lancet alignment is maintained, and vibration and play in the drive mechanism are reduced.

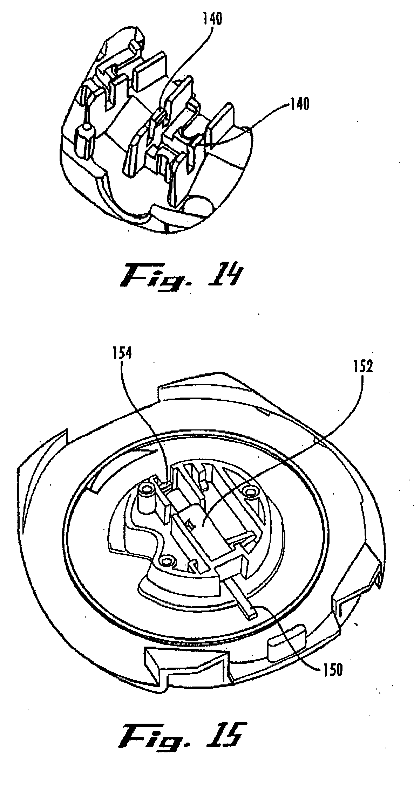

For example, **FIGURE 15** shows an anti-rotation interlock mechanism for a multi-lancet lancing device, comprising an arm **150** projecting from the distal end of the drive piston **152,** opposite the lancet engaging jaw **154.** The arm engages a cooperating recess or other surface feature of the lancet cartridge when the drive mechanism is cocked, to prevent motion of the cartridge, but to allow advancement of the cartridge after firing.

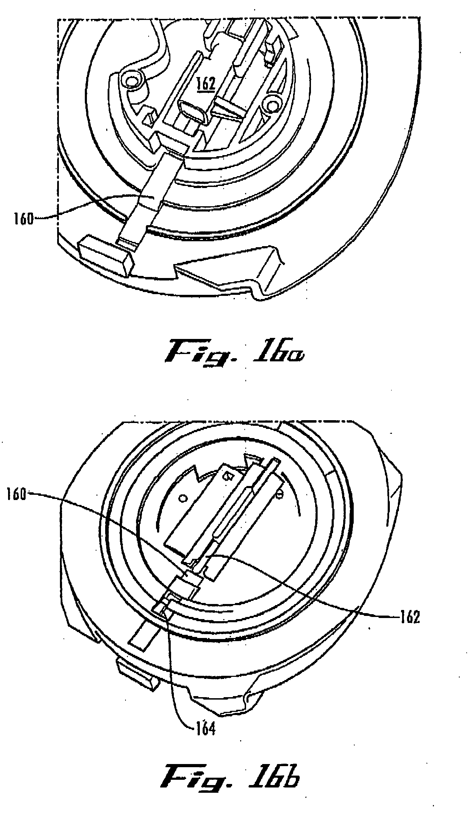

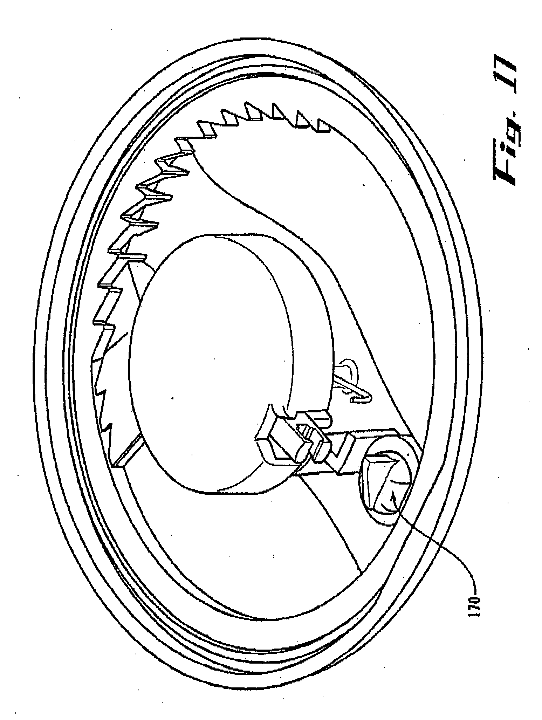

In other example embodiments, such as shown in **FIGURES 16a** and **16b**, the anti-rotation interlock features comprise a locking bolt **160**, which is retracted upon contact with the drive piston **162** when the drive mechanism is cocked, to engage the lancet cartridge and lock it in position. A projection **164** on the lower face of the locking bolt **160** interfaces with a cooperating feature on the advancing mechanism to block further advancement of the cartridge and thereby prevent double-cocking and/or potential jamming. In other embodiments, the device includes a ratcheting retaining ring for retaining the lancets in place and for preventing double-cocking and/or re-use by preventing rotation of the lancet cartridge when the device is cocked.

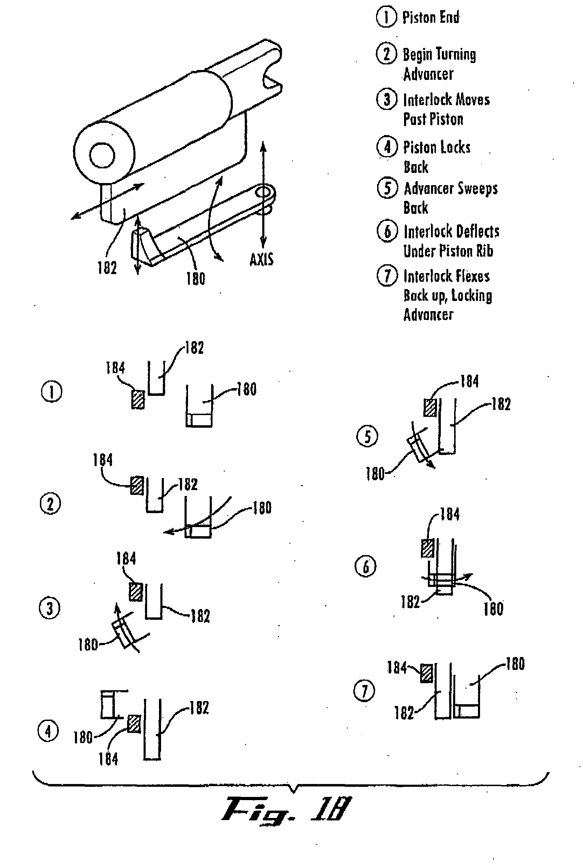

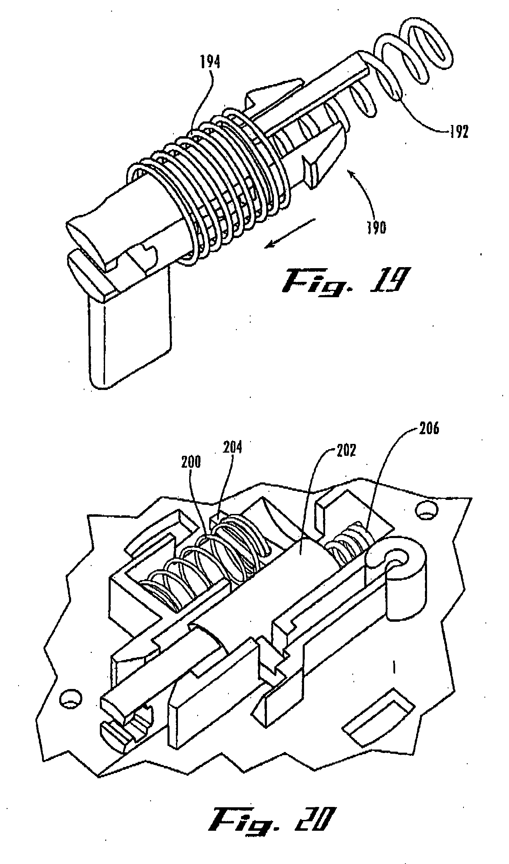

**FIGURE 17** shows an embodiment of the invention including a spring-biased plunger **170** having an inclined shoulder for engagement with cooperating detents in the lancet cartridge to index the cartridge as it is advanced through sequential lancets of the cartridge, and to prevent reverse movement of the cartridge.

The multi-lancet cartridge of the present invention optionally also includes a break-away section that differentiates a new cartridge from a used cartridge, to prevent accidental re-use of a potentially contaminated lancet. For example, a flag or indicator can be provided for manual displacement by the user, or which is automatically broken off of the cartridge or otherwise displaced upon insertion into the housing or upon initial advancement or firing. In its various embodiments, the cartridge can be assembled using assembly methods including one or more of: ultra-sonic welding, snaps, crush pins, solvent bonding, adhesive, thermal welding, and/or laser welding.

### Drive Mechanism and Actuation

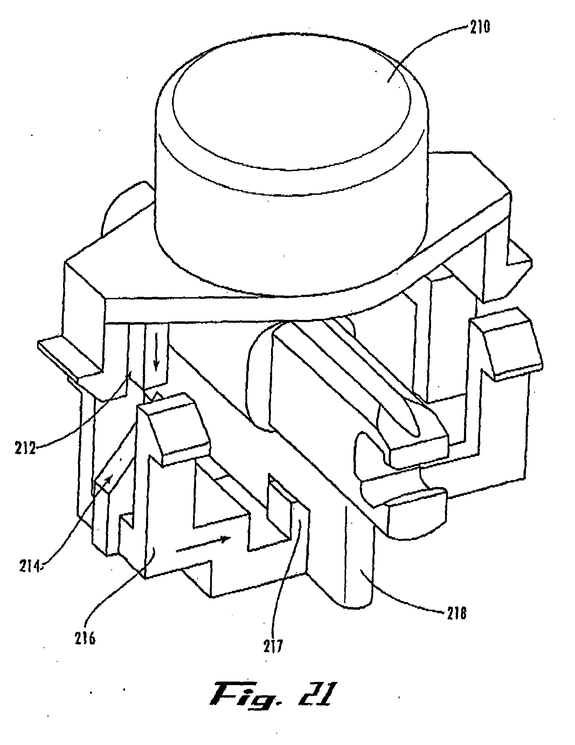

Example embodiments of the lancing device of the present invention preferably also include an improved drive mechanism, and/or an improved actuation (i.e., trigger) mechanism. For example, **FIGURE 18** shows a portion of a drive mechanism for a multi-lancet lancing device according to an example embodiment of the invention, including a cantilevered flexing interlock spring arm **180** for engagement with a cooperating interlock fin **182** on the drive piston, and a re-inforcement rib **184** in the mechanism base, to prevent double-cocking of the lancing device, thereby reducing the likelihood of jamming. In the depicted sequence of operation, the piston **182** is in its forward (fired) position when the user begins turning the advancer. The interlock arm **180** moves past the piston, and the piston locks in its rearward (charged) position. The advancer is then moved back along its return stroke, and the interlock arm **180** deflects under the piston **182**. The interlock 180 then flexes back up, locking the advancer. The piston is charged, and the reinforcement rib **184** prevents the interlock fin of the piston **182** from bending.

**FIGURE 19** shows a drive mechanism including an in-line piston assembly 190, having one of the drive spring **182** or return spring **194** externally mounted thereon, abutting against an exterior shoulder; and the other of the drive or return springs internally mounted within a bore in the distal end of the piston, in a coaxial nested manner. The opposed springs operate in tandem to advance and retract the piston (and the active lancet coupled thereto) through its lancing stroke.

**FIGURE 20** shows another embodiment of a drive mechanism, including a laterally offset retraction spring **200** alongside the drive piston **202**, and in the plane of the array of lancets in or on the cartridge. The retraction spring **200** operates against a laterally projecting arm **204** of the piston **202.** The drive spring **206** is in line with the piston **202,** and operates against the distal end of the piston.

**FIGURE 21** shows an improved trigger mechanism according to an example embodiment of the invention. Actuation of the release button **210** drives a finger **212** connected to the button along an inclined surface **214** of a sliding cage **216,** moving the cage transversely, and moving a sear surface **217** out of engagement with the drive piston **218** to release the piston and fire the device.

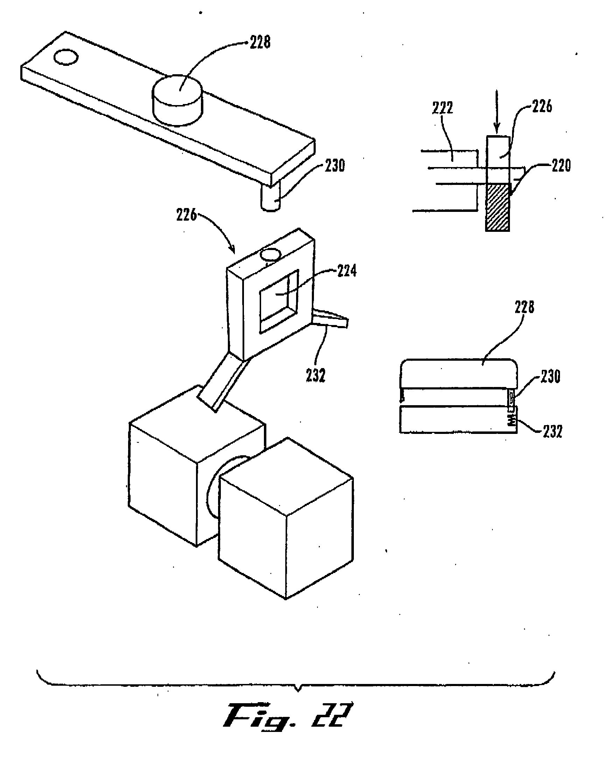

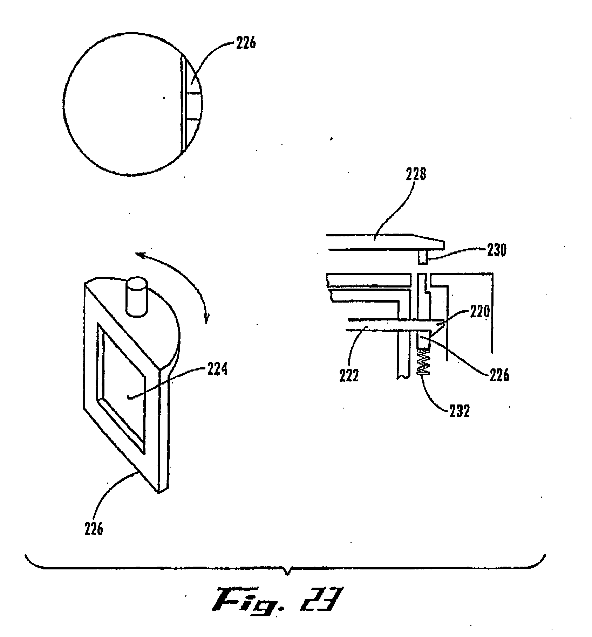

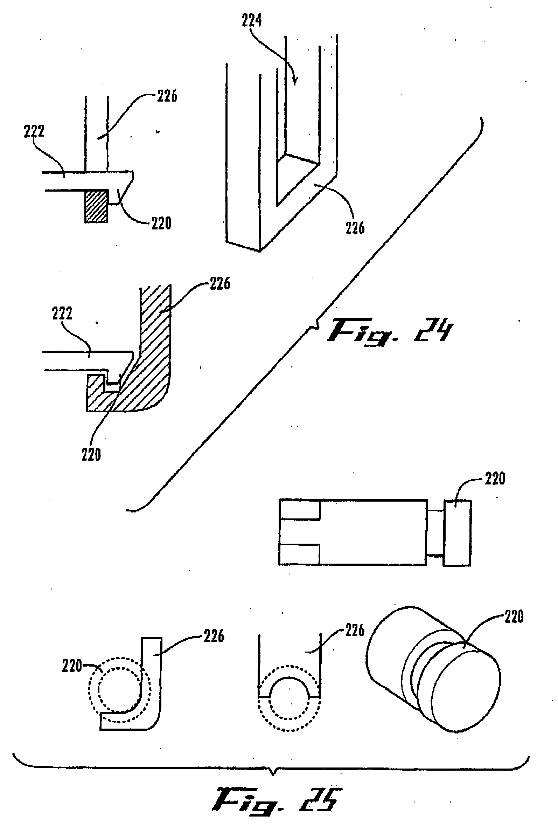

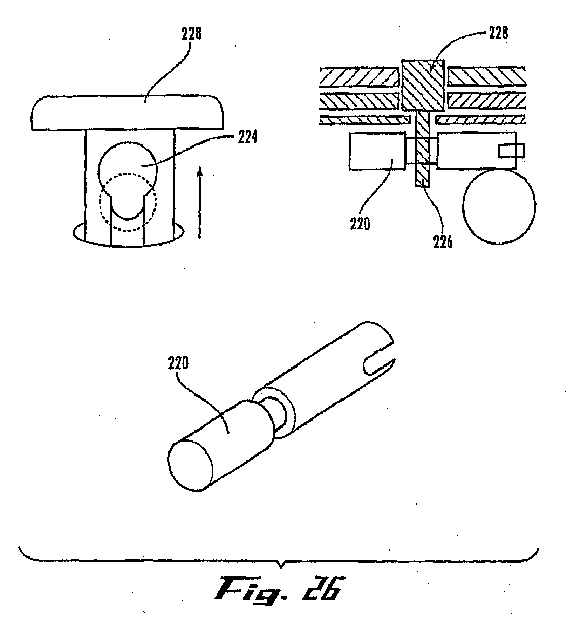

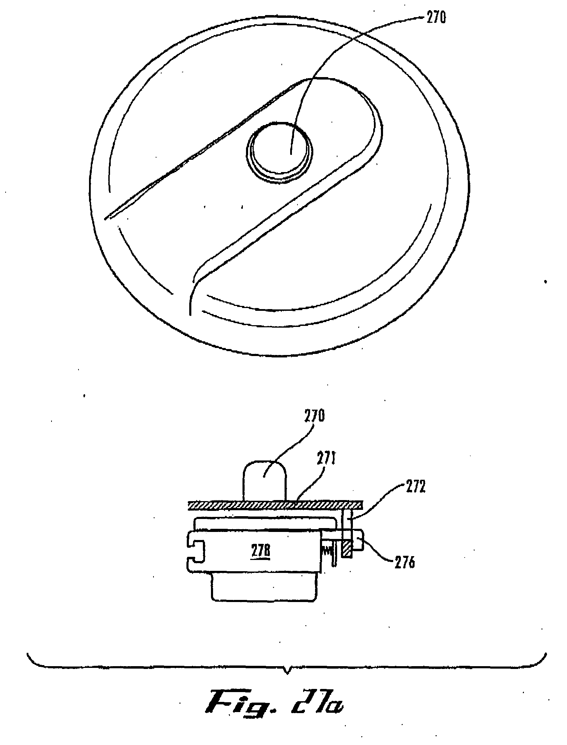

**FIGURES 22 - 26** show various alternate embodiments of shutter trigger mechanisms according to example forms of the invention. In general, a barb or expanded portion **220** extending from the drive piston **222** releasably engages within an aperture **224** formed in a shutter or release member **226.** The release member **226** is actuated to release the barb or expanded portion **220** from engagement with the aperture **224** to fire the lancing device, in the embodiment of **FIGURE 26,** a keyhole aperture **224** includes a large diameter portion allowing passage of expanded portion **220** therethrough, and a smaller diameter portion for engagement with a portion of the drive piston having a reduced diameter. An actuator button **228** or other member is preferably provided external of the lancing device housing, with a finger or other projection **230** extending through the housing into contact with the release member to actuate the device. One or more spring members **232** are preferably provided to return the shutter to the ready state after firing.

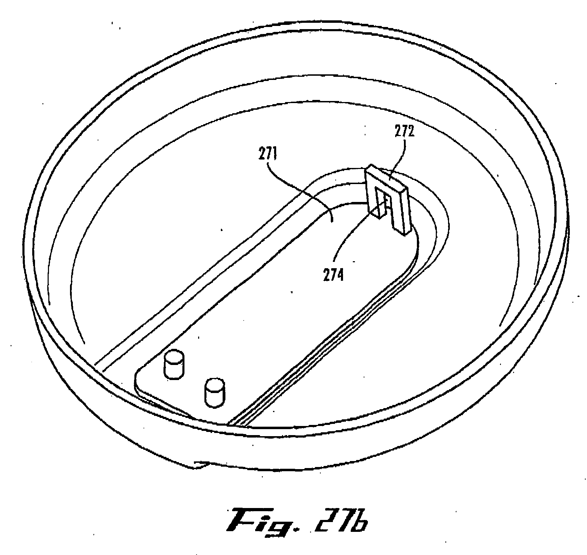

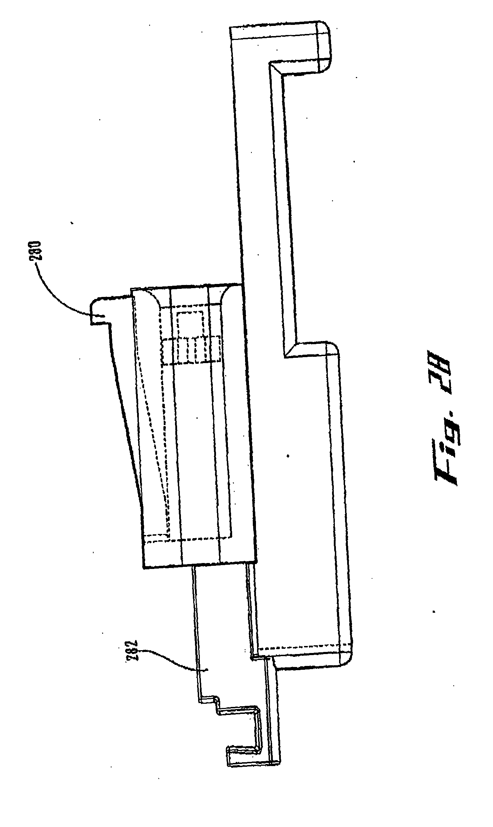

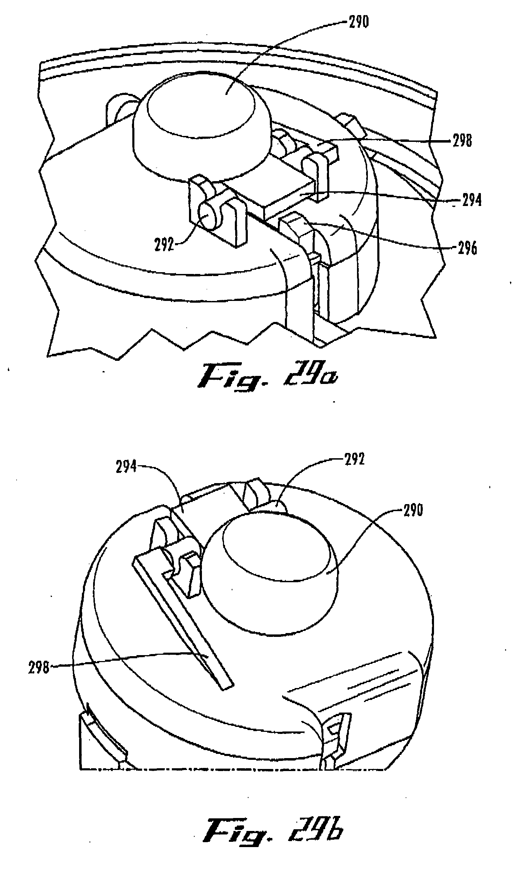

**FIGURES 27a** and **27b** show a flexing trigger button mechanism having an actuator button **270** mounted to a flexing arm **271**, coupled to a release member **272** having an aperture **274** formed therein for releasably engaging a barbed or angled arm **276** projecting from the distal end of the drive piston **278.** Pressing the button **270** moves the release member **272** out of engagement with the arm **276** to release the piston **278** and fire the device. In the embodiment of **FIGURE 28,** a trigger release member **280** is integraly molded with the drive piston **282**.

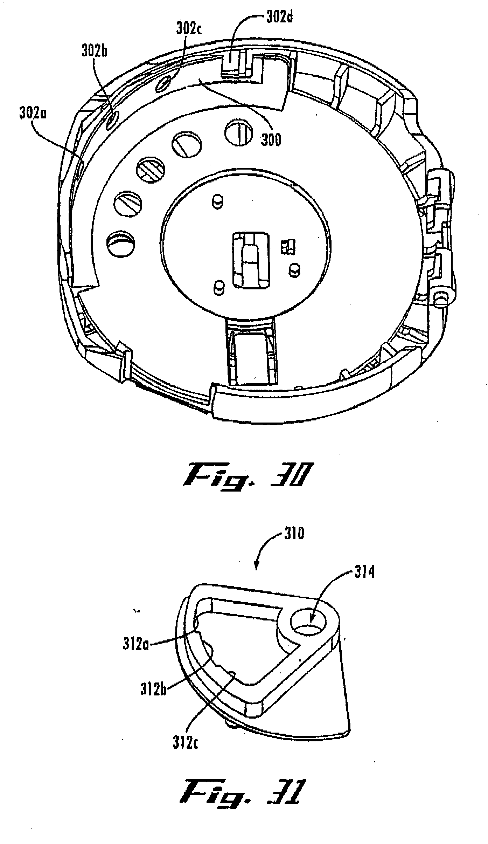

**FIGURES 29a** and **29b** show a hinged trigger mechanism having a release button **290** on one side of a fulcrum **292,** and a release or sear surface **294** on the opposite side of the fulcrum, Pressing the button **290** downward raises the sear surface **294** out of engagement with a cooperating trigger arm **296** projecting from the drive piston, releasing the drive piston to fire the device. A return spring **298** is preferably unitarily molded into the trigger mechanism.

In other embodiments, the device includes a trigger molded into the top cover of the lancet cartridge. For example, a trigger button and release arm may be integrally molded with the top cover, as by forming at least a portion thereof of a flexible material. In still other alternate embodiments, a cantilevered trigger arm extends alongside the drive piston, and has a free end in releasable engagement with a cooperating surface of the drive piston. The free end of the trigger arm is flexed out of engagement with the drive piston to fire the device.

### Depth Adjustment

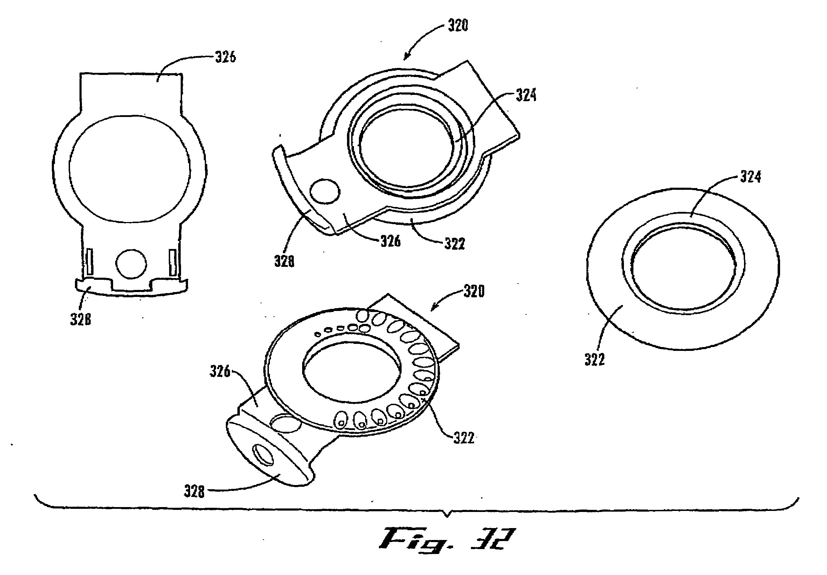

Example embodiments of the lancing device of the present invention preferably also include improved depth control features for enabling the user to selectively vary the depth of penetration of the lancet tip into the skin at the lancing site. For example, **FIGURE 30** shows a depth control mechanism according to an example embodiment of the invention, having a rotating plate **300** with multiple openings **302** therethrough for providing lancing depth adjustment. Depth adjustment can be accomplished, for example, by providing openings of different depth and/or diameter through a wall of the plate **300,** by forming the wall of the plate to have different thicknesses at different points along its length, and/or by forming the wall to be radially offset by differing amounts at different angular positions. Rotation of the plate within the housing of the lancing device, for example by means of an external actuator member, brings the desired opening into alignment with the lancet opening through the housing.

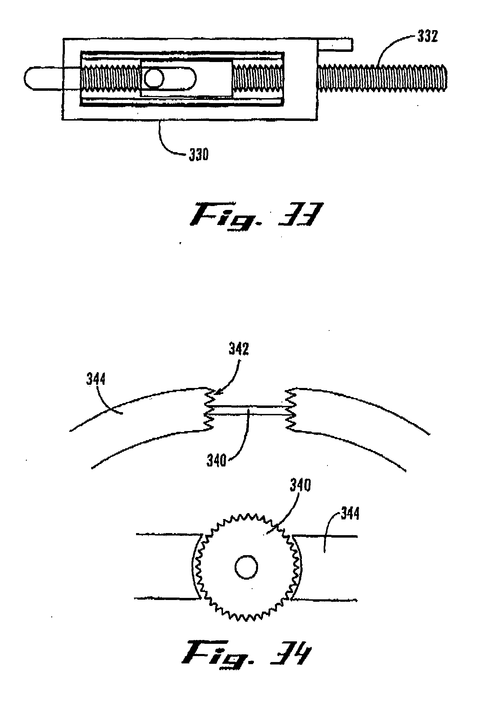

**FIGURE 31** shows a pivotal stroke-limiting depth control stop **310.** The lancet impacts a generally arcuate contact face having a stepped surface with a plurality of stroke-limiting surfaces **312,** each providing a different penetration depth. The user pivots the depth stop about an axis **314** to position the contact face with the selected portion of its stepped surface in line for contact with the active lancet to select the desired lancing depth.

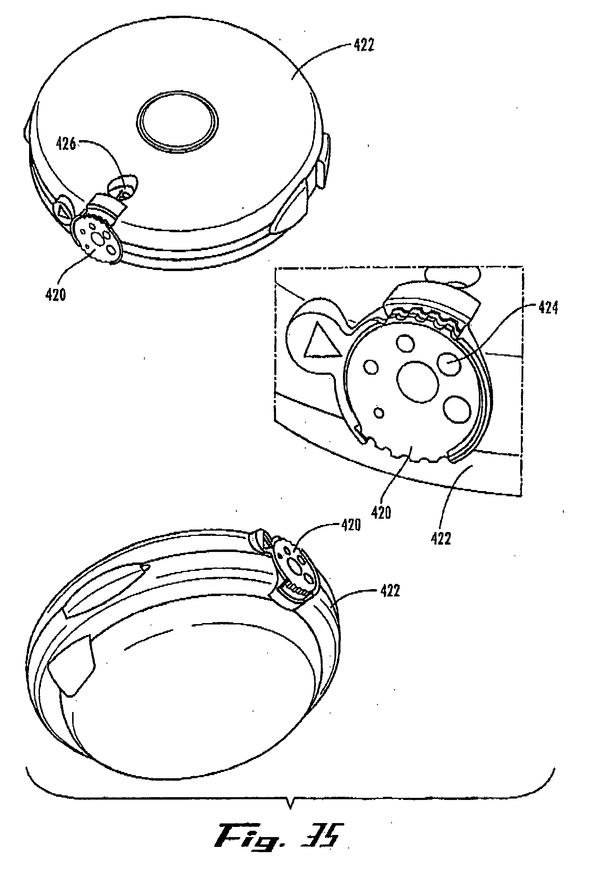

**FIGURE 32** shows a sliding plate depth-control mechanism **320** with a dial member **322** having an eccentric hub **324** rotationally mounted within a cooperating opening of a translationally sliding plate member **326.** Rotation of the dial **322** adjusts the position of a contact face **328** surrounding the lancet opening of the lancing device housing, to vary the depth of penetration.

In alternate embodiments, replaceable lancet cartridges are provided in different "sizes" for providing different lancing depths. For example, cartridges can be sold in "shallow", "medium" and "deep" sizes, and the user purchases the desired size. The cartridges are interchangeable for use with a standard lancing device, and the variation in depth can be provided, for example, by varying the lancet needle length, the wall thickness, etc. In still other embodiments, a positional adjustment mechanism such as a screw-driven rack is provided for varying the position of the cartridge and/or the drive mechanism within the housing of the lancing device. In still other embodiments, the position or spring constant (stiffness) of the return spring and/or the drive spring of the lancet drive mechanism can be varied to provide depth control,

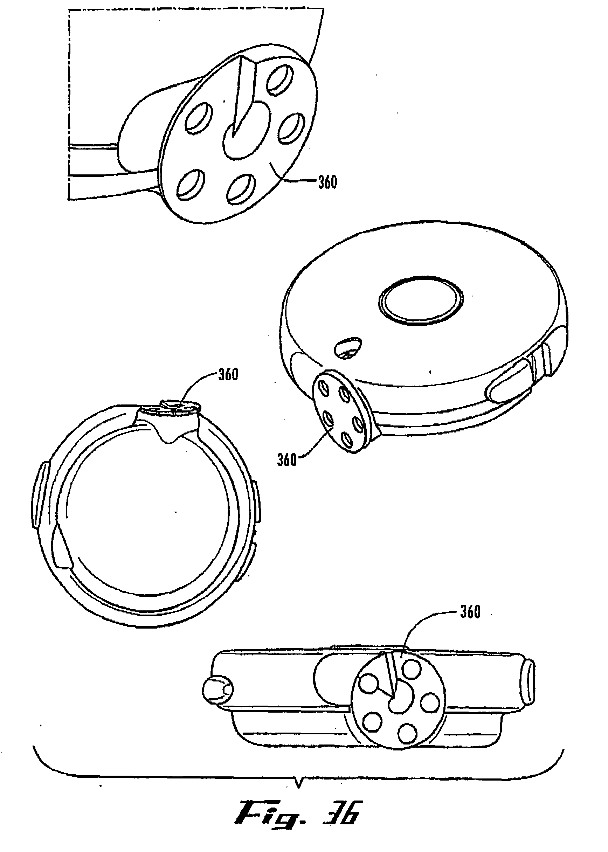

**FIGURE 33** shows a depth control mechanism incorporating a movable throttle plate **330** having front and back stops that move in tandem via actuation of a positioning screw **332** to provide depth adjustment.

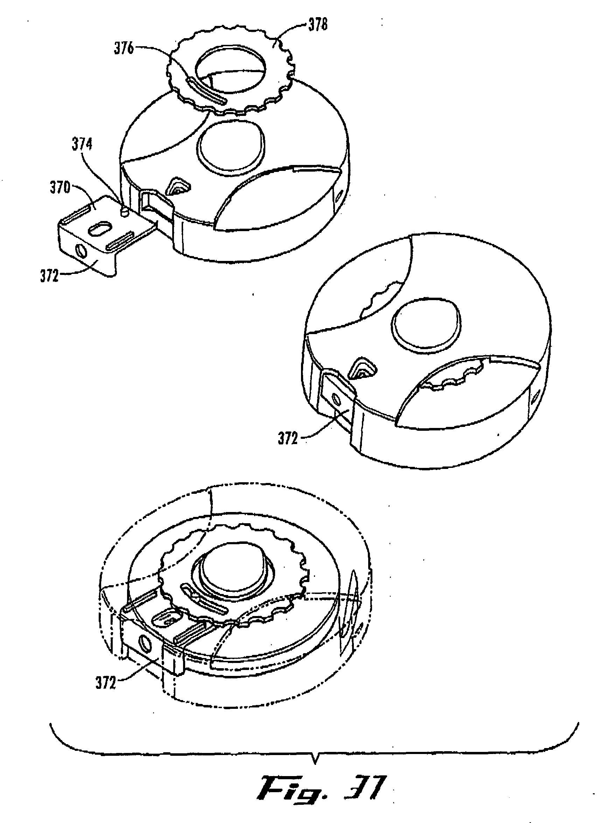

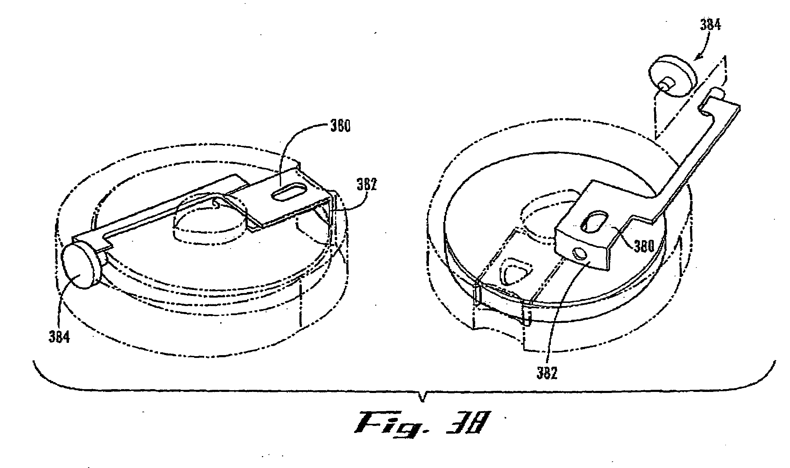

The depth control mechanism of **FIGURE 34** includes a circular threaded plate **340** with a lancet opening through its center, mounted within a cooperatively threaded opening **342** in the lancet device housing **344.** The plate is turned to screw it in and out relative to the wall of the housing, providing a variable inner contact surface for limiting the stroke of the lancet, and/or a variable depth recess surrounding the lancet opening, for lancing depth control.

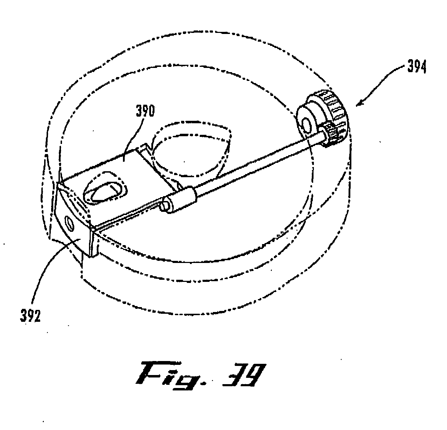

**FIGURE 35** shows a depth control mechanism comprising a circular depth wheel **420** rotationally mounted to the housing **422,** and having a plurality of lancet openings **424** of differing diameter angularly offset from one another. The user rotates the wheel **420** to bring a selected one of the lancet openings into alignment with the path of travel of the active lancet, thereby varying the penetration depth. Numerical or other indicia **426** on or coupled to the depth wheel **420** are optionally provided, visible from the exterior of the housing, to inform the user of the selected penetration depth.

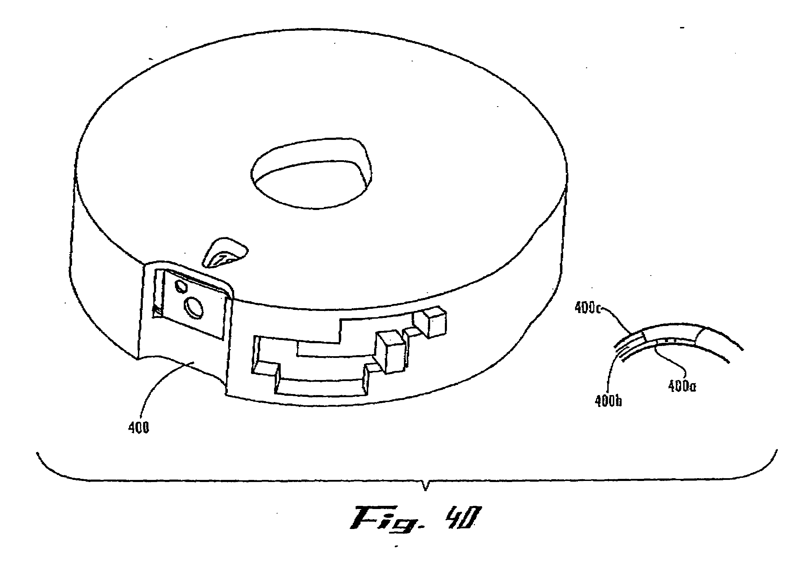

**FIGURE 36** shows a depth control mechanism comprising a tapered-thickness depth wheel **360** rotationally mounted to the housing. The depth wheel defines a plurality of angularly offset lancet openings, and has a wall thickness that varies about its circumference (i.e., different thicknesses at different angular displacements around the disk), The varying wall thickness allows the user to select the desired lancing depth by rotating the depth wheel to bring a selected one of the lancet openings into alignment with the path of travel of the active lancet.

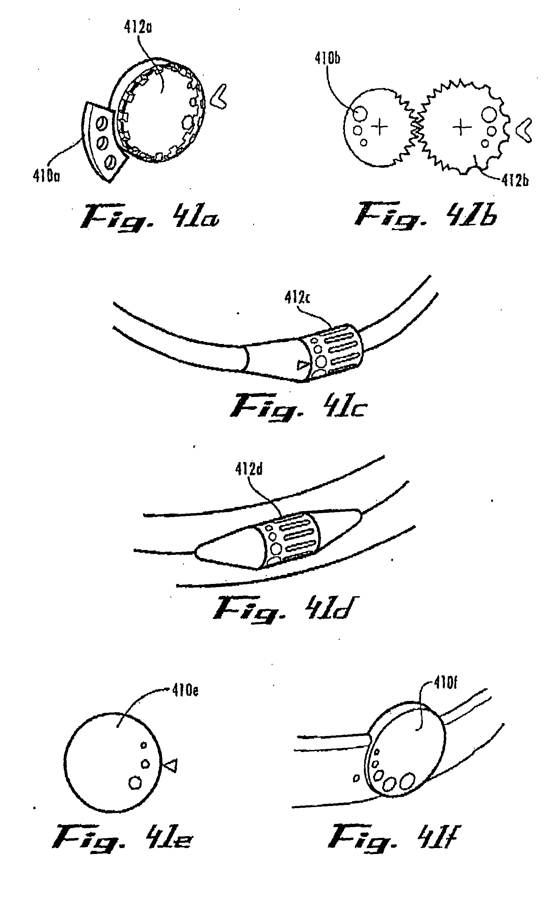

The depth control mechanism of **FIGURE 37** comprises a sliding plate **370** with an adjustable-position contact face **372** for placement against the lancing site to vary the depth of penetration. A pin **374** on the plate is engaged within an eccentricly arcuate cam slot **376** of a depth-adjustment wheel **378,** which the user rotates to vary the position of the contact face relative to a lancet stroke-limiting surface of the housing, to adjust the penetration depth.

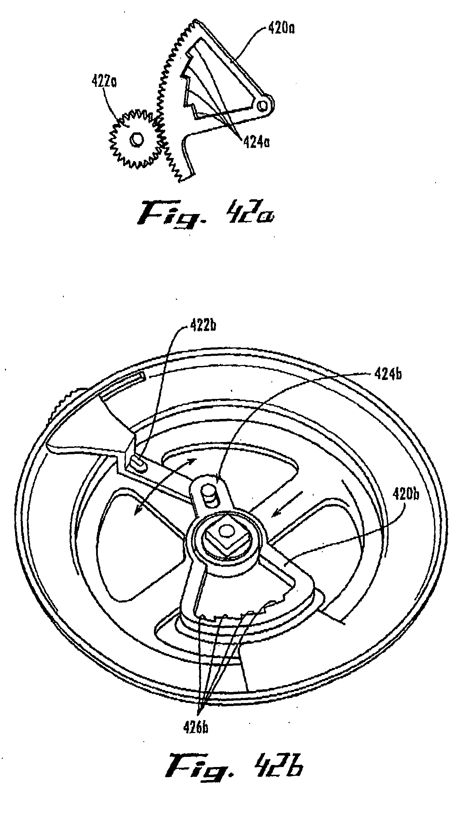

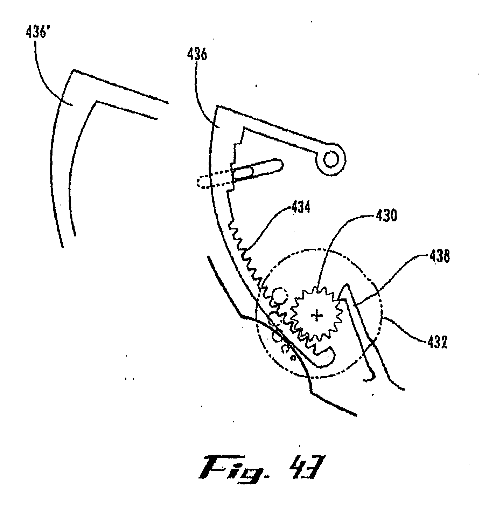

**FIGURE 38** shows a depth control mechanism having a sliding plate 380 with an adjustable-position contact face **382** for placement against the lancing site to vary the depth of penetration from lancing. A screw-drive mechanism **384** provides adjustment of the position of the contact face. **FIGURE 39** shows a similar depth control mechanism having a sliding plate **390** with an adjustable-position contact face **392** for placement against the lancing site to vary the depth of penetration from lancing. A gear-driven threaded rod **394,** with an end engaged in a threaded sleeve portion of the sliding plate 390, provides adjustment of the position of the contact face.

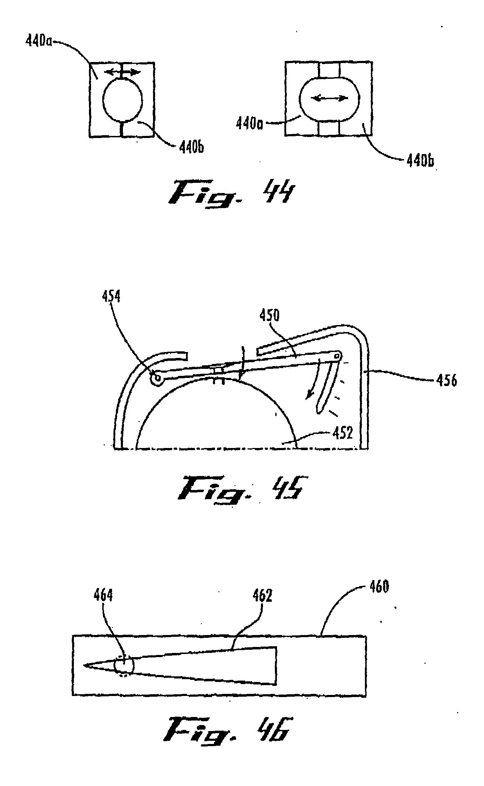

**FIGURE 40** shows a depth control mechanism having one or more shutters **400** for varying the effective wall thickness of the housing of the lancet device to control lancing depth. Each shutter has a lancet opening therethrough, and can be moved between a first position wherein its lancet opening is aligned with the path of travel of the active lancet, and a second position away from the path of travel of the active lancet. As successive shutters are moved to their respective first positions, their cumulative thickness increases the spacing between the forward face of the lancet body and the lancing site, thereby decreasing the penetration depth. Adjacent shutters are radially offset from one another, so that one, two, or more of the shutters can be selectively opened or closed to vary the lancing depth.

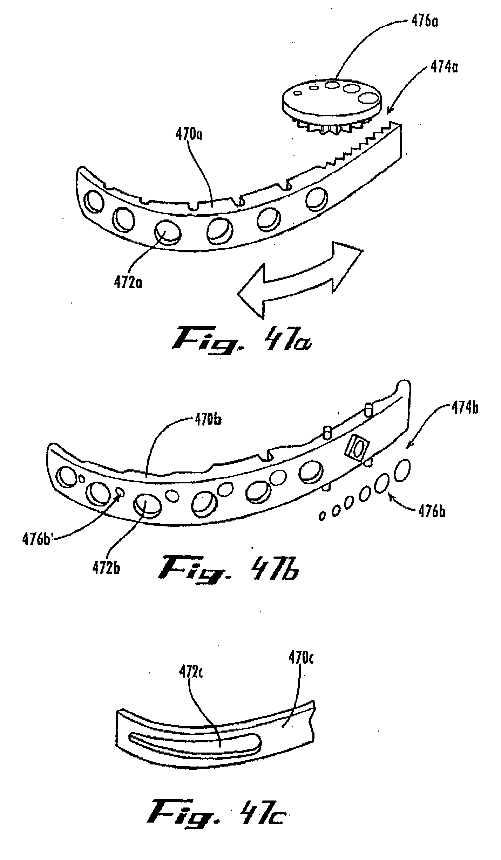

The depth control mechanism can comprise a depth control member **410** that is rigidly attached or integrally formed with an adjustment member **412**, as shown for example in **FIGURE 41a**, wherein an inclined depth control member having a varying thickness along its length projects radially from the adjustment knob. Alternatively, the depth control mechanism comprises separate depth control and adjustment members directly or indirectly coupled by gearing or other linkage means, as shown for example in **FIGURES 41b-41f.**

The angular position of the pivotal depth control member **420a** of **FIGURE 42a** is adjusted via gear drive **422a** to selectively position one of the stepped contact faces **424a** to limit the stroke of the lancet. The angular position of the pivotal depth control member **420b** of **FIGURE 42b** is adjusted via a toggle ling age **422b** having a sliding pivot joint **424b,** to selectively position one of the stepped contact faces **426b** to limit the stroke of the lancet.

The depth control mechanism of **FIGURE 43** includes an axial spur gear **430** mounted to the adjustment knob **432,** driving a toothed track **434** to align a selected contact face portion of the depth control member **436** to limit the stroke of the lancet and thereby control penetration depth. The depth control member can comprise a stepped contact face (**436**), or an inclined contact face (**436'**). A detent cantilever **438** is optionally provided, contacting the toothed surface of the spur gear **430** for indexing and tactile feedback to the user.

The depth control mechanism of **FIGURE 44** comprises an adjustable-width sliding iris having opposed halves **440a, 440b** that can be moved closer to or further away from one another to reduce or increase the opening size, thereby varying the extent to which the subject's skin may bulge into the opening to vary penetration depth. A larger opening size allows the skin of the lancing site to be received further therein for deeper lancing, and a smaller opening size providing shallower lancing. **FIGURE 45** shows a depth control mechanism having a pivoting contact plate **450,** the position of which is angularly variable relative to the position of the lancing cartridge **452,** by means of a hinged connection **454** to the housing **456,** to adjust the depth of penetration.

**FIGURE 46** shows a depth control mechanism having a contact face **460** for placement against the skin at the lancing site, the contact face comprising a tapered slot **462**, wider at one end than at the other. Adjustment of the position of the tapered slot relative to the lancet opening **464** varies the effective opening size, thereby varying the depth of penetration.

**FIGURE 47** shows a depth control mechanism having a flexible sliding band **470** defining a contact face for placement against the skin at the lancing site, and selectively movable across the housing In front of the housing's lancet opening. The thickness of the band **470** varies along its length, and/or the dimension of the opening(s) **472** through the band varies, to provide lancing depth adjustment. A geared adjustment knob **474a,** or pin-and-detent slide coupling **474b** provide positional adjustment of the band **470** relative to the housing. The geared face of the adjustment knob **474a** can operate vertically on a toothed surface on the front or back face of the band as shown, or can operate horizontally on a toothed surface on the top or bottom edge of the band. Depth indicators can be provided on or adjacent the adjustment knob (**476a**) or the slide coupling (**476b**); and/or along the band (**476b'**) for viewing through a window adjacent the openings. Optionally, the band **470** can be moved completely out of the way of the lancet opening through the housing, to provide maximum lancing depth, as for alternate site lancing. In alternate forms, the band slides within the housing, behind a contact surface for placement against the skin at the lancing site, to limit the stroke of the lancet and/or to vary the opening size, and thereby control penetration depth.

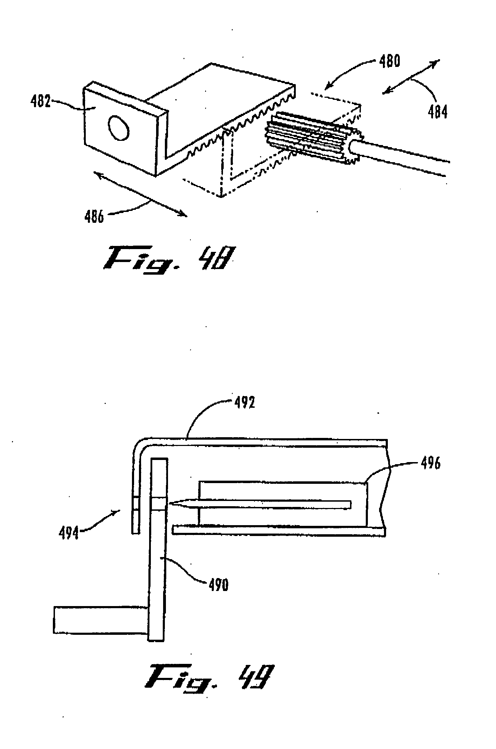

The depth control mechanism of **FIGURE 48** comprises a rack-and-pinion drive mechanism **480** for advancing and retracting the position af a sliding plate **482** along a first axis (indicated by directional arrow **484**), the sliding plate having a contact face for contact with the skin at the lancing site, to adjust lancing depth, Optionally, the sliding plate **482** can slide along a second axis (indicated by directional arrow **486**), perpendicular to the first axis, to move the plate out of the path of the active lancet for maximum penetration depth, as for alternate site lancing.

**FIGURE 49** shows a depth control mechanism having a depth adjustment insert member **490** with a flange selectively insertable or removable to and from the device housing **492** adjacent the lancet opening **494**, to limit the stroke of the lancet **496** and thereby vary depth of penetration.

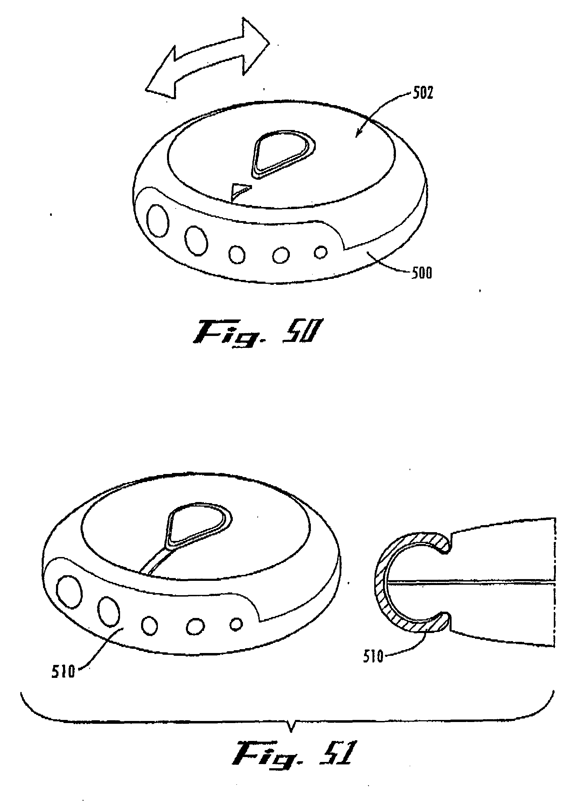

**FIGURE 50** shows a depth control mechanism having an outer housing **500** with openings of different sizes, and an inner mechanism **502** rotationally mounted within the housing to select one of the openings for alignment with the active lancet, and thereby control lancing depth. **FIGURE 51** shows a depth control mechanism having an external sliding plate **510** with varying wall thickness and/or opening sizes, which is selectively movable along the housing to align one of the openings with the active lancet and thereby vary the lancing depth. The sliding plate **510** optionally serves also as a latch to secure the upper and lower housing halves to one another.

**FIGURE 62** shows a depth control mechanism having a continuous depth adjustment ring-shaped wall **520** rotationally captured between the lancing device mechanism base **522** and the bottom enclosure **524** of the housing **528.** The ring-shaped wall **520** has varying depth-control opening sizes and/or wall thickness to control lancing depth. The ring-shaped wall **520** preferably extends circumferentially within the disk-shaped housing **528** adjacent an inner surface **532** of a housing exterior sidewall **530** and around the cartridge **12** and the lancing device mechanism base **522.** The ring-shaped wall **520** is rotatable relative to the housing **528** and the cartridge **12** to a plurality of positions. In each of the positions a different one of the depth-control openings **534a, 534b** .. **534n** (collectively the dopth-control openings **534a-n**) is aligned with an active one of lancets **16** and the lancing opening **536** of the housing **528** for receiving the active lancet therethrough during its lancing stroke. In the depicted embodiment, the ring-shaped wall **520** has a uniform thickness and two end segments **520a** and **520b** that overlap and align to cooperatively define the depth-control openings **534a-n**, with one of the end segments radially offset by differing amounts relative to the housing **528** when the wall is in the different rotational positions for adjusting the lancing penetration depth. One or more portions of the ring **520** are accessible from the exterior of the housing **628** through opening(s) **526** in the housing, to permit rotational adjustment of the ring-shaped wall **520** by the user.

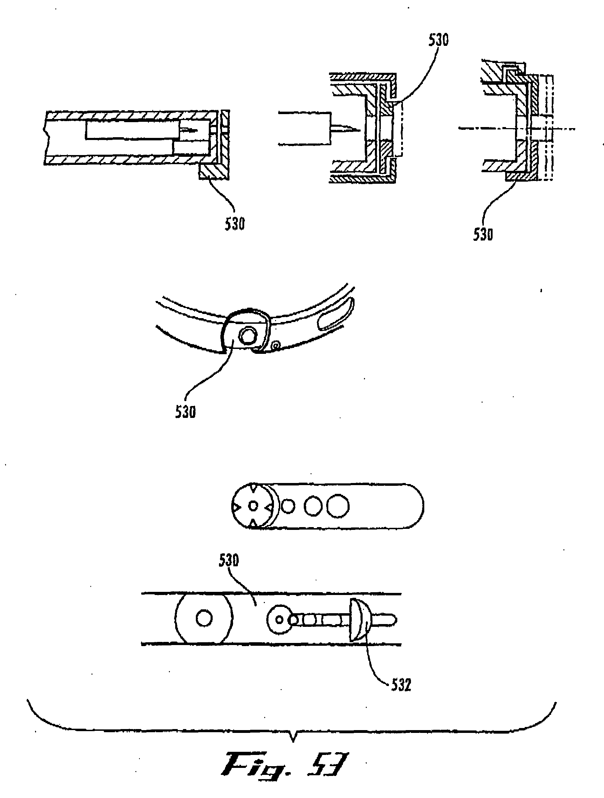

**FIGURE 53** shows a depth control mechanism having a depth adjustment band **530** movably mounted along the outside of the housing, and having varying opening sizes and/or wall thickness to control lancing depth. A sliding adjustment knob **532** is preferably provided for controlling the positioning of the depth adjustment band **530**.

While the invention has been described with reference to preferred and example embodiments, it will be understood by those skilled in the art that a variety of modifications, additions and deletions are within the scope of the invention, as defined by the following claims.

## Claims

1. A lancing device (10) for use in combination with a multi-lancet cartridge (12) comprising a plurality of lancets (16) for lancing skin, the lancing device comprising:
a housing (528) that receives therein the multi-lancet cartridge (12), having an exterior sidewall (530) defining a lancing opening;
a drive mechanism operable to propel an active one of the lancets through a lancing stroke to penetrate the skin; and
an advancing mechanism operable to sequentially advance the lancets (16) of the multi-lancet cartridge (12) sequentially into engagement with the drive mechanism; the device being
**characterised by**
a depth-control mechanism (28) providing adjustment of a depth of the penetration into the skin of the active lancet (16) during its lancing stroke, the depth-control mechanism (28) including a wall (520) that is curved, rotationally mounted to the housing (528), interposed between the cartridge (14) and the housing sidewall (530), and defines a plurality of depth-control openings(534a, 534b, ..., 530n), wherein the wall (520) is rotatable relative to the housing (528) and cartridge (12) to a plurality of positions, wherein in each of the positions a different one of the depth-control openings (534a, 534b, ..., 530n) is aligned with the active lancet (16) and the lancing opening of the housing for receiving the active lancet therethrough during its lancing stroke.

2. The lancing device of claim 1, wherein the wall (520) is ring-shaped and extends circumferentially within the housing (528) adjacent an inner surface of the housing exterior sidewall (530).

3. The new device of Claim 2, wherein the wall (520) has two end segments that overlap and align to co-operatively define the depth-control openings(534a, 534b, ..., 530n), with one of the end segments radially offset by differing amounts relative to the housing when the wall (520) is in the different rotational positions for adjusting the lancing penetration depth.

4. The lancing device of Claim 1, wherein the wall (520) has a uniform thickness.

5. The lancing device of Claim 1, wherein in the different rotational positions the wall (520) is radially offset by differing amounts relative to the housing for adjusting the lancing penetration depth.

6. The lancing device of Claim 1, wherein the housing (528) defines a control opening (526) and a portion of the wall (520) is accessible through the housing control opening (526).

7. A lancing device (10) for use in combination with a multi-lancet cartridge (12) comprising a plurality of lancets (16), the lancing device comprising a housing, a drive mechanism, and an advancing mechanism for bringing the lancets (16) of the multi-lancet cartridge 912) sequentially into engagement with the drive mechanism, the device being **characterised by** a depth-control mechanism (28) providing adjustment of a depth of penetration of an active one of the lancets.
